# EUROPEAN PATENT SPECIFICATION

(11) **EP 1 609 431 B1**
(45) Date of publication and mention of the grant of the patent: **24.12.2008**
(21) Application number: 05253881.6
(22) Date of filing: 22.06.2005
(51) Int. Cl.: A61B 19/00

(54) **Haptic device for use in surgical simulation systems**
Vorrichtung mit taktiler Rückkopplung zur Verwendung in chirurgischen Simulationssysteme
Dispositif à perception tactile pour utilisation dans des systemes de simulation chirurgicale

(30) Priority: 22.06.2004 US 872395
(43) Date of publication of application: 28.12.2005
(73) Proprietor: Simsurgery AS, 0855 Oslo (NO)
(72) Inventor: Rotnes, Jan Sigurd, 1415 Oppegard (NO); Westgaard, Geir, 1412 Sofiemyr (NO); Sorhus, Vidar, 1410 Kolbotn (NO); Eriksen, Eivind Myrold, 0568 Oslo (NO); Overskeid, Oyvind, 1405 Langhus (NO)
(74) Representative: Robson, Aidan John

(56) References cited:
- EP-A- 1 391 181
- EP-A- 1 543 789
- WO-A-03/096307
- FR-A- 2 719 760

## Description

The invention is related to a haptic device for use in a surgical simulation system.

The closest prior art is EP-A-1391181, which discloses a haptic device suitable for use in a surgical simulation system as defined in the preamble of claim 1.

A number of studies around the world suggest that approximately 10% of patients admitted to the hospital suffer some kind of harm, about half of which is preventable with current standards of treatment. Although the majority of these adverse events are minor, some lead to serious injury or death. A significant percentage of these adverse events is associated with a surgical procedure. In the United Kingdom, complication rates for some of the major operations are 20-25% with an acceptable mortality of 5-10%. However at least 30-50% of major complications occurring in patients undergoing general surgical procedures are thought to be avoidable.

Furthermore, new trends in modem medicine are the development of information technology and image processing, allowing minimally-invasive, image-guided therapy, either as an interventional radiological technique or as video-assisted surgery e.g. laparoscopy. In the treatment of some diseases, such as gall-bladder disease, peripheral vascular or coronary stenosis, minimally-invasive techniques are already applied in the majority of cases. It is likely that this trend will continue when fully digitized imaging modalities further evolve. These new procedures require significant training since the technology imposes both new possibilities and limitations regarding surgical instruments and how these are controlled. Computer based simulators have been shown to improve performance in the operative theatre (faster and less errors).

In order to provide better patient care with new technology and methods, the medical profession faces significant challenges in education and training since adoption to new procedures often require hands-on experience. Today surgeons are learning technical skills in the operating room using the 100 years old Halstedian principle "see one, do one, teach one".

The possibility of using virtual reality simulators in surgical training was proposed more than a decade ago. This form of training holds the potential of reducing the need for mechanical models and animals in surgical training without compromising surgical outcome. Mechanical trainers are being used to train and evaluate laparoscopic surgical skills, but compared to virtual reality simulators the assembly of mechanical trainers is time consuming. After each session mechanical trainers have to be reassembled and prepared again for the next student, and they do not allow automated measurements of surgical performance. However, with the continuously increasing speed of computers, surgical simulators are now being offered to hospitals as agents to improve training and reduce cost of education. Computer based simulators will increasingly be more eligible as a training aid, especially due to their extensive assortment of educational features as:
- Individual digital proctors can serve as an integrated part of the simulator based on skills and ambitions of the students. The (virtual) individual proctor can besides providing relevant training scenarios also report progress and comparative information.
- The quality and effectiveness of carrying out a simulated procedure can be recorded, and the data can form valuable feedback by analyzing the instrument trajectories and visualizing the results e.g. simulating an angiographic sequence after a coronary bypass.
- The clinical diversity experienced in real life due to differences in anatomy, different pathological processes and stages, requires a large and flexible model basis that is probably only possible with a computer based simulator.
- The purchase expense of phantom based training systems is most likely to be lower than it is for computer based systems, but the total cost of ownership with much use may be higher. Training with phantom entails risk of instruments breakage.
- Training with computer based simulators is a novel approach with versatile pedagogic potentials that might motivate physicians to improve their skills and better assess new techniques.
- Several computer based trainers might be connected in a network that renders an effective way to maintain and update the systems. Furthermore, a database that includes recordings from the training sessions might provide valuable statistical information.
- New medical technologies like surgical robots also demand innovation in design of new instruments and visualization systems. Production, testing and distribution of such prototypes are expensive which might exclude good ideas to be tried out. However, by introducing a virtual model in a computer based simulator important feedback might be provided from the users to the designers before physical prototyping and testing - virtual "beta-testing".
- Video database, 3D-anatomical atlas, multimedia presentations and videoconferences from "live" procedures may run on the same digital platform as the computer based simulators, and thereby enhance the quality and extend the content of training sessions.

Use of computer based simulators is closely related to the provision of satisfactory interface devices.

US5623582 describes a computer interface or control input device for laparoscopic surgical instrument and other elongated mechanical objects, ie an apparatus for interfacing the movement of a shaft with a computer. The apparatus includes a support, a gimbal mechanism having two degrees of freedom, and three electromechanical transducers. This device provides the user with several degrees of freedom with respect to moving the surgical instruments, but the instruments still have limited possibilities of movement, and will not give the user the impression of a real surgical situation. The device comprises also complicated mechanical parts which are prone to wear and are expensive to repair.

Other prior art simulating techniques employ ordinary surgical instruments which are inserted into an operative cavity where the movement of the instruments are filmed by a camera and shown on a screen. The simulations require physical imitations of the body parts, and real sutures etc., to be able to carry out the simulation. This limits the range of possible surgical operations and makes the simulation less realistic.

At the Simulation and Visualization Research Group at University of Hull, there has been developed a Virtual Environment Knee Arthroscopy Training System. This system comprises a pair of mock instruments (arthroscope camera and surgical probe) which is used in conjunction with a hollow, articulated model of a knee. The system further comprises a tracking system for tracking the orientation and position of the instruments and for showing simulated views on a computer screen. The orientation and position of the instrument is tracked with an electromagnetic tracking system which is also used to measure movement of the knee joint. This system is a dedicated knee arthroscopy training system and can not be used for simulating other types of surgical procedures. The model of the knee has an appointed hole for inserting the instruments and the surgeon can thus not decide the position himself.

The object of the invention is to provide a haptic device for use in surgical simulators that gives the physical impression of a real surgical situation without being confined to mechanical devices and without need for sensors on the patient mannequin.

It is a further object of the invention to provide an operating element and haptic device for use in surgical simulators which give a realistic physical environment for the simulation. It is a further object of the invention to provide an operating element and haptic device that may be used for several different surgical procedures..

A haptic device according to the invention is defined in claim 1 and may be used with an operating element adapted for receiving haptic devices and/or surgical instruments/devices.

The operating element comprises an operating surface adapted for insertion of surgical instruments and/or devices. The operating surface may be a flat surface, a curved surface, or may have any desired shape. In one embodiment, the operating element is formed to have the shape of a part of the human body to give the user a more realistic impression of a real operation. The surface of the operating element may comprise different textures depending on which body part that is imitated or depending on the user's wishes and needs.

The operating element may also have different shapes depending on which type of operation to simulate. In case of laparoscopic simulations, the operation element will be a flat or otherwise shaped plate with holes, the holes simulating the ports/pivot points where the tools/instruments are inserted in the surgical procedure. In open surgical simulations, the operation element will be shaped to imitate the cavity or shape of the open body part in question.

In the laparoscopic simulation case, the holes in the operation element may be arranged in patterns that are adequate for the specific surgical procedure. In some embodiments, there may be only few holes arranged in the typical locations for that procedure, to provide the user with the correct positions for surgery. In other embodiments, the operation element may be a more generic element, with a number of holes suitable for several different procedures or operation types, or to enable the user to choose the appropriate location for the specific operation. Further embodiments may have holes arranged in patterns, e.g. circular patterns or matrix, and the user may choose the holes to use, or can be provided with a "map" showing the correct or suggested holes for different surgical procedures. The latter hole configuration is particularly useful for simple, general, flat operating surfaces which not necessarily imitate a body part, but may be used for several different simulated surgical procedures. Using one such a generic operating surfaces, the simulating system may be very simple, but still has the ability of simulating a wide range of different surgical procedures of different part of the body.

The operating element may in a further embodiment comprise a support device, e.g. a frame or any other structure to make the operating element rigid. The operating element or the support device may also comprise legs. The legs may in one embodiment of the invention be adjustable to adapt the height of the operating element to the user. The operating element may comprise clamping device for fixing the operation element to a table or other surface or environment where the simulation is to be performed. The clamping device may be a clamp, for example of the type used for clamping drawing board lamps to a table, or may be a suction device or any other suitable clamping or fastening means.

The operating element may also comprise a pad connected to the operating surface for simulating different thickness of the patient's body.

The haptic device is the instrument manipulation apparatus used in surgical simulations. The haptic device in a racing game could be a joystick or a steering wheel, while the haptic device of a laparoscopic simulator is mostly specially designed tools that mimic the rack of tools involved in laparoscopy. The haptic device may be a copy of a real surgical tool, a real surgical tool more or less adapted to use in a simulating system, or a dedicated simulation tool.

The haptic device according to the invention comprises a handle part, an instrument part, an adapter releasable connected to the handle part and the instrument part and comprising at least one motion tracking sensor, and a transmission part for transmission of sensor signals to the simulation system.

The handle part may be a generic handle for use in different types of operations, or the handle part may be shaped as a surgical instrument and/or may have the functionality of a surgical instrument. In one embodiment, the handle part comprises a rotary wheel and a sensor for detecting the angular orientation of the wheel. The function of the rotary wheel is to change the angular orientation of the instrument part without having to rotate the handle part and thus the hand holding the handle part. The sensor detects the angular (rotational) orientation of the wheel and transmits the sensor signal to the simulation system which interprets the signal as a rotation of the instrument part and shows the rotation on the screen of the simulation system.

The instrument is in its simplest embodiment a rod. The length and diameter of the rod is adapted to a real surgical instrument/tool. The objective of the instrument part is to provide a mechanical coupling to the operating element and thus provide a realistic environment for the simulations. The system can be used without the instrument part comprised in the haptic device, but it will then be a less realistic handling of the haptic device.

The adapter is adapted for connecting to the handle part and the instrument part. The connection may be any suitable connection, e.g. swan socket, click-fit connection, etc, and can be adapted to a standard handle connection or a dedicated connection.

The adapter may also comprise means for detecting and transferring information regarding the manipulation of the handle, e.g. information regarding closing or opening of a grip. When the handle part comprises a rotary wheel, the sensor for detecting the angle and/or the means for transmitting the sensor signals may be comprised in the adapter.

In another possible embodiment of the haptic device, the haptic device comprises a handle part, and preferably an instrument part. The motion tracking sensor and/or the handle manipulation detector can be a separate sensor unit for connection to the handle part. The sensor unit may also comprise transmission means for transmitting motion tracking sensor signals and/or handle manipulation signals to the simulating system.

The operating element and the haptic devices can interact, but they may also be used as independent devices. The haptic devices may be used without any operating element, or with another adequate physical interface. The purpose of the operating element is to provide a realistic working environment for the surgeon, and it does not comprise a tracking system itself. The operating element is thus independent of the haptic devices and may be used with any chosen motion tracking system for the haptic devices. It is possible to arrange a tracking system in the operating element, but this will in principle be substantially independent from the mechanical constraints provided by the element.

The motion tracking sensor is a sensor for tracking the position and movement direction of the haptic device. The sensor signals are transmitted to the simulating system where they are processed and applied to the imaging of the tool on a screen. In this way, the user sees his manipulation of the instrument/tool directly on the screen as in the real operational situation.

The motion tracking sensor may be any motion tracking sensor able to track both position and movement of the instrument with adequate resolution. The motion tracking sensor may e.g. be part of an electromagnetic tracking system, ultrasound tracking, mechanical tracking, etc. It is also possible to use a combined tracking system with different sensors for position and direction, e.g. a position tracking by means of microwaves combined with a gyroscope for sensing the direction.

The motion tracking sensor is preferably connected to the adapter and may be integrated in the adapter.

The signal transmission means will transmit the signals from the motion tracking sensors of the haptic devices of the simulation system. The signal transmission means may be wireless, or the signals may be transferred by means of wires to the simulation system. The simulation system will comprise means for processing the received signals and will integrate the information into the simulated images shown on the user's screen. It is also possible that the motion tracking sensors comprise processing means for processing the sensor signals and adapt the signals for use directly in the simulation system.

In the case where the motion tracking sensors need a reference for being able to precisely define the correct position in space, the reference information also should be transferred to the simulation system. When the operating element and the haptic devices are part of a common system, the operating element constitutes a physical reference which should be reflected in the simulations. This may be done by arranging a reference unit in the operating element and transmitting the reference signal to the simulation system. The reference signals may be transferred by means of the same or different signal transmission means as those used for transmitting the motion tracking sensor signals.

The invention will now be described in more detail by means of examples with reference to the accompanying figures.
Figure 1 shows an overview of a simulation system comprising the kit according to the invention.
Figure 2a, 2b and 2c shows three embodiments of an operating element according to the invention.
Figure 3a and 3b shows one embodiment of a haptic device according to the invention.
Figure 4a and 4b shows another embodiment of a haptic device according to the invention.
Figure 5 show an overview of another embodiment of a simulation system comprising a kit according to the invention.

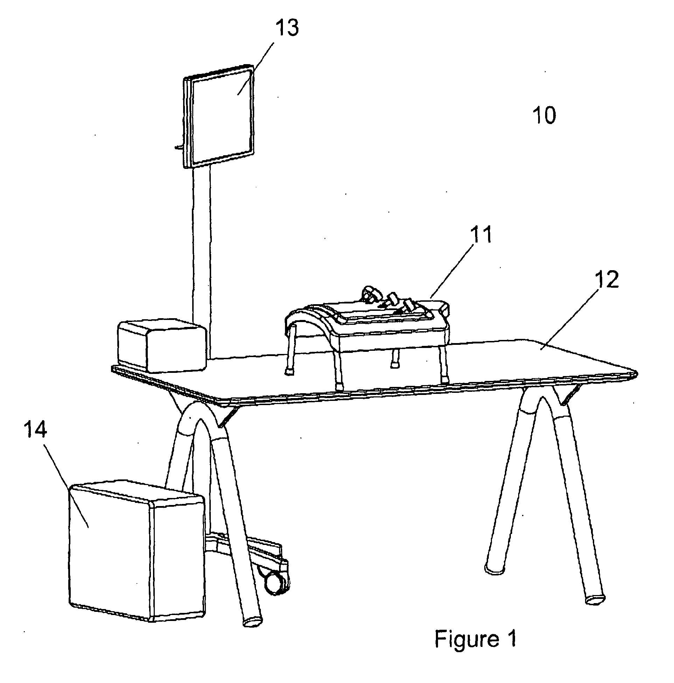

Figure 1 shows an overview 10 of a simulation system for surgical simulations. The system comprises a kit according to the invention comprising an operating element 11, which constitutes a patient mannequin, standing on a table 12 and haptic devices (not shown) which correspond to surgical instruments/tools/devices of a real operation. The signals regarding movement and manipulation of the haptic devices are processed by a computer 14 and shown on a screen 13 in real time, thus giving the user the impression of a real operating situation.

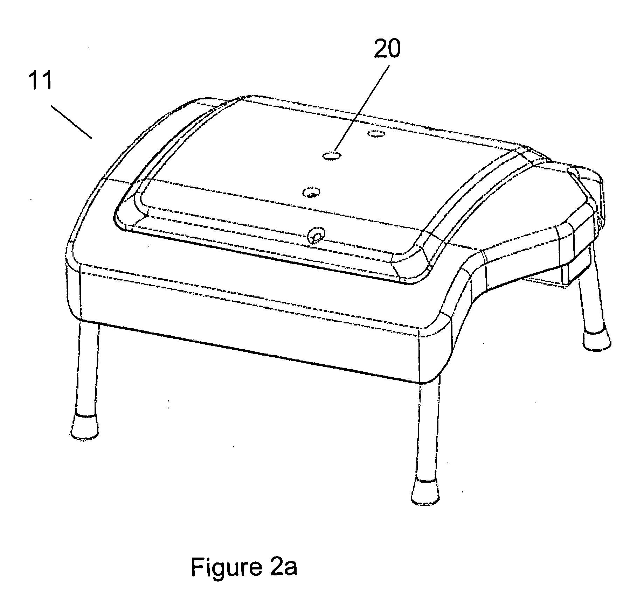

Figure 2a shows an embodiment of an operating element 11 according to the invention for simulating laparoscopic operations. The operating element is in this embodiment curved, thus e.g. resembling an abdomen, and has 5 holes 20 for inserting the haptic devices. The holes 20 may be adapted for the specific procedure which is to be simulated. In one simulation procedure, some or all of the holes may be used. The holes 20 constitute pivot points and thus the physical interface corresponding to the patient's body.

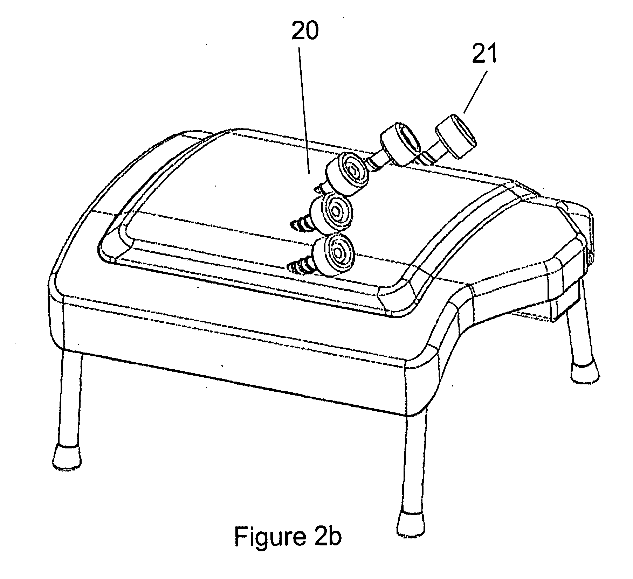

In figure 2b trocars 21 are mounted in the holes of the operating element, further enhancing the user's impression of a real procedure. The trocars 21 may be standard trocars, or they may have a simplified design for simulating purposes.

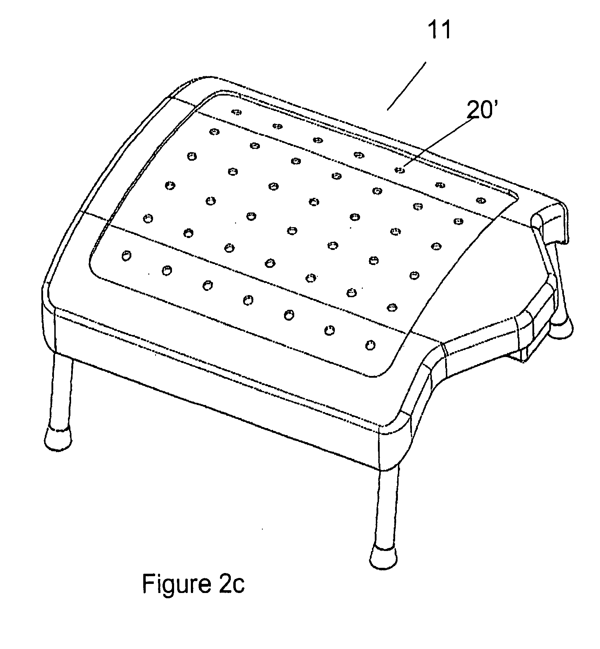

Figure 2c shows an embodiment of an operating element 11 with a matrix of holes 20' in it. This operating element may be used for a number of different surgical procedures by employing different hole "coordinates" for the different procedures.

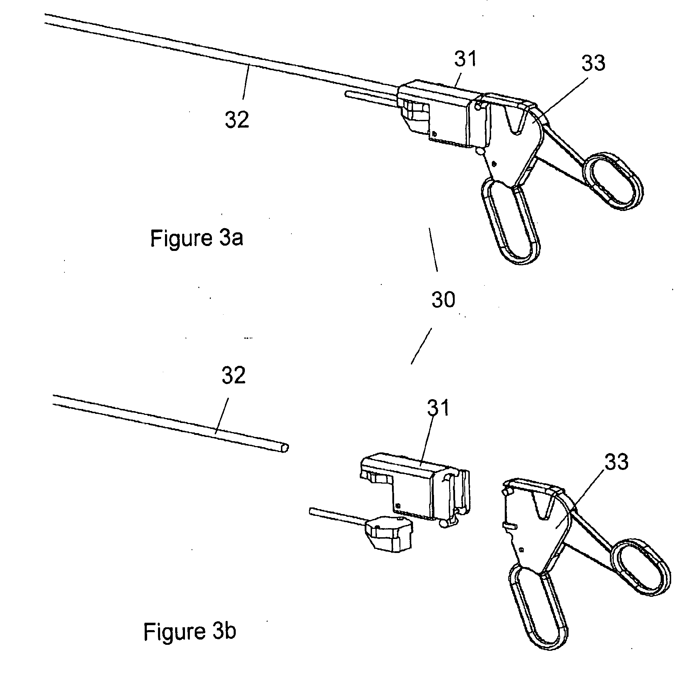

Figure 3a and 3b show an embodiment of a haptic device 30 according to the invention. Figure 3a shows the haptic device 30 in assembled view, and figure 3b shows the haptic device in exploded view. In the exploded state, the separate parts of the device can easily be recognised. The haptic device comprises an adapter part 31, an instrument part 32 and a handle part 33. The adapter part further comprises a motion tracking sensor device 34 for sensing the position and movement direction of the haptic device 30. The motion tracking sensor is in this example a part of a motion tracking system of Polhemus, Vermont, USA. The Polhemus system comprises a device for applying a magnetic field in the tracking area. The motion tracking sensors comprise several coils arranged in different positions/directions and when the coils are moved, the currents induced by the magnetic field change and this provides the motion tracking signals. In one embodiment, the device for applying the magnetic field is arranged on the operating element.

The handle part 33 and the instrument part 32 are adapted for connection to the adapter part 31. As can be seen from the figure, the connection between the handle part and the adapter part is a click-fit connection which enables the user to easily change the handle part.

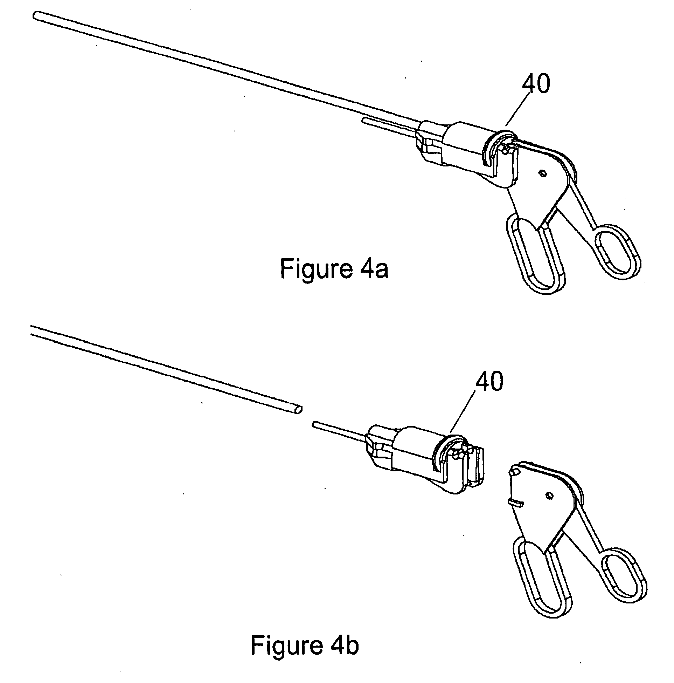

Figures 4a and 4b show another embodiment of the haptic device according to the invention. In addition to the features shown in figure 3, this embodiment comprises a rotary wheel 40. The rotary wheel 40 corresponds to similar arrangements in real surgical tools, and when the user rotates this wheel, a signal is transferred to the simulating system's processing equipment. When the user rotates the wheel, this then results in a rotational movement of the surgical tool imaged on the screen of the simulation system, and the user thus have access to the full functionality of a real surgical tool.

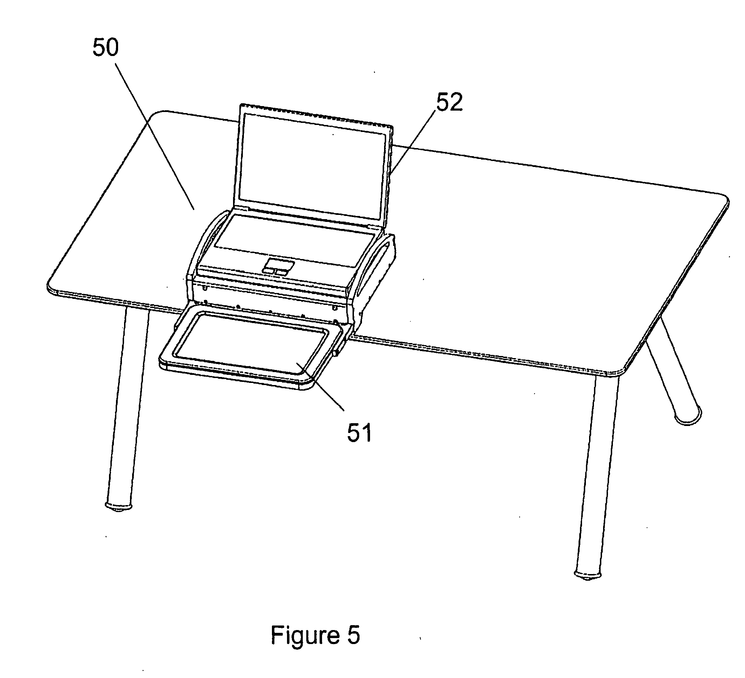

Figure 5 shows different views of another embodiment of a simulation system comprising a kit 50 according to the invention. This embodiment constitutes a very compact and portable system which may be used in different locations. The operating element 51 is in this embodiment shaped as a flat plate and supported by a frame. The frame is connected to a housing which houses a laptop computer 52 for processing the signals regarding movement and manipulation of the haptic devices and showing the results on the screen in real time. The frame may be disconnected and put on top of the laptop computer 52 for transport or storage, or the frame may be arranged to be inserted into the housing when not in use. As mentioned above, the flat plate may be equipped with holes.

## Claims

1. Haptic device for use in a surgical simulation system, the haptic device comprising:
- handle part (33),
- an instrument part (32) adapted for insertion in an operating surface of an operating element (11),
- at least one motion tracking sensor (34), and
- a transmission part for transmission of sensor signals to the simulation system,
**characterised by** an adapter (31) releasably connected to the handle part (33) and the instrument part (32), said adapter (31) comprising said at least one motion tracking sensor (34).

2. Haptic device according to claim 1, wherein the transmission part is integrated in the adapter (31).

3. Haptic device according to claim 1, wherein the handle part (33) comprises a rotary wheel (40) and a sensor for detecting the angular position of the wheel, and means for transmission of sensor signals.

4. Haptic device according to claim 1, wherein the handle part (33) is substantially shaped as a surgical instrument.

5. Haptic device according to claim 1, wherein the handle part (33) has the functionality of a surgical instrument.

6. Haptic device according to claim 1, including
an operating element (11) comprising:
- an operating surface adapted for insertion of surgical instruments and/or haptic devices,
- a support device for supporting the operating surface.

7. Haptic device according to claim 6, wherein
- the operating element (11) comprises a number of holes (20) for inserting surgical tools or haptic devices.

8. Haptic device according to claim 6, wherein the operating element (11) is shaped as part of a human body.

9. Haptic device according to claim 6, wherein the haptic device also comprises a trocar (21) releasably arranged in the operating element (11).

10. Haptic device according to claim 6, wherein the operating surface is shaped as part of a human body.

11. Haptic device according to one of the claims 6-10, wherein the operating surface is a plane surface.

12. Haptic device according to one of the claims 7-11, wherein the holes (20) are arranged in a pattern.

13. Haptic device according to one of the claims 7-11, wherein the holes (20) are arranged in a matrix.

14. Haptic device according to claim 6, wherein the operating surface is curved.

15. Haptic device according to one of claims 6-14, wherein the support device comprises legs.

16. Haptic device according to claim 15, wherein the legs are adjustable.

17. Haptic device according to any one of claims 6-16, wherein the support device comprises clamping devices for fixing the operating element to a table.

18. Haptic device according to one of claims 7-17, wherein the haptic device comprises a trocar releasably arranged on some of the holes.

19. Haptic device according to any one of claims 6-18, wherein the operating surface comprises a pad.

## Patentansprüche

1. Haptische Vorrichtung zur Benutzung in einem chirurgischen Simulationssystem, wobei die haptische Vorrichtung umfasst:
- einen Griffteil (33),
- einen Instrumententeil (32), welcher zum Einführen in eine Operieroberfläche eines Bedienelements (11) angepasst ist,
- zumindest ein Bewegungsverfolgungssensor (34), und
- einen Übertragungsteil zur Übertragung von Sensorsignalen an das Simulationssystem, **dadurch gekennzeichnet, dass** ein Adapter (31) entfernbar mit dem Griffteil (33) und dem Instrumententeil (32) verbunden ist, wobei der Adapter (31) zumindest einen Bewegungsverfolgungssensor (34) umfasst.

2. Haptische Vorrichtung nach Anspruch 1, worin der Übertragungsteil in einen Adapter (31) integriert ist.

3. Haptische Vorrichtung nach Anspruch 1, worin der Griffteil (33) ein Drehrad (40) und ein Sensor zum Detektieren der Winkelstellung des Rads und Mittel zur Übertragung von Sensorsignalen umfasst.

4. Haptische Vorrichtung nach Anspruch 1, worin der Griffteil (33) im Wesentlichen als chirurgisches Instrument geformt ist.

5. Haptische Vorrichtung nach Anspruch 1, worin der Griffteil (33) die Funktionalität eines chirurgischen Instruments hat.

6. Haptische Vorrichtung nach Anspruch 1, ein Bedienelement (31) umfassend, welches umfasst:
- eine Operieroberfläche, angepasst zum Einführen von chirurgischen Instrumenten und/oder haptischen Vorrichtungen,
- eine Unterstützungsvorrichtung zum Unterstützen der Operieroberfläche.

7. Haptische Vorrichtung nach Anspruch 6, worin das Operierelement (11) eine Anzahl von Löchern (20) zum Einführen von chirurgischen Werkzeugen oder haptischen Vorrichtungen umfasst.

8. Haptische Vorrichtung nach Anspruch 6, worin das Operierelement (11) als Teil eines menschlichen Körpers geformt ist.

9. Haptische Vorrichtung nach Anspruch 6, worin die haptische Vorrichtung auch einen Trokar (21) umfasst, welcher entfernbar in dem Operierelement (11) angeordnet ist.

10. Haptische Vorrichtung nach Anspruch 6, worin die Operieroberfläche als Teil des menschlichen Körpers geformt ist.

11. Haptische Vorrichtung nach einem der Ansprüche 6 bis 10, worin die Operieroberfläche eine ebene Oberfläche ist.

12. Haptische Vorrichtung nach einem der Ansprüche 7 bis 11, worin die Löcher (20) in einem Muster angeordnet sind.

13. Haptische Vorrichtung nach einem der Ansprüche 7 bis 11, worin die Löcher (20) in einer Matrix angeordnet sind.

14. Haptische Vorrichtung nach Anspruch 6, worin die Operieroberfläche gebogen ist.

15. Haptische Vorrichtung nach einem der Ansprüche 6 bis 14, worin die Unterstützungsvorrichtung Beine umfasst.

16. Haptische Vorrichtung nach Anspruch 15, worin die Beine einstellbar sind.

17. Haptische Vorrichtung nach einem der Ansprüche 6 bis 16, worin die Unterstützungsvorrichtung Befestigungsvorrichtungen zum Befestigen des Operierelements an einem Tisch umfasst.

18. Haptische Vorrichtung nach einem der Ansprüche 7 bis 17, worin die haptische Vorrichtung einen Trokar umfasst, welcher entfernbar auf einigen der Löcher angeordnet ist.

19. Haptische Vorrichtung nach einem der Ansprüche 6 bis 18, worin die Operieroberfläche ein Kissen umfasst.

## Revendications

1. Dispositif à perception tactile, pour utilisation dans un système de simulation chirurgicale, le dispositif à perception tactile comprenant :
- une partie formant poignée (33),
- une partie formant instrument (32), adaptée pour insertion dans une surface d'actionnement d'un élément d'actionnement (11),
- au moins un capteur de suivi de déplacement (34), et
- une partie de transmission, pour la transmission de signaux de capteur au système de simulation,
**caractérisé par** un adapteur (31), connecté de manière désolidarisable à la partie formant poignée (33) et à la partie formant instrument (32), ledit adaptateur (31) comprenant ledit au moins un capteur de suivi de déplacement (34).

2. Dispositif à perception tactile selon la revendication 1, dans lequel la partie de transmission est intégrée dans l'adaptateur (31).

3. Dispositif à perception tactile selon la revendication 1, dans lequel la partie formant poignée (33) comprend une roue (40) rotative et un capteur, pour détecter la position angulaire de la roue, et des moyens pour transmission des signaux de capteur.

4. Dispositif à perception tactile selon la revendication 1, dans lequel la partie formant poignée (33) est conformée sensiblement en instrument chirurgical.

5. Dispositif à perception tactile selon la revendication 1, dans lequel la partie formant poignée (33) a la fonctionnalité d'un instrument chirurgical.

6. Dispositif à perception tactile selon la revendication 1, incluant :
un élément d'actionnement (11), comprenant :
- une surface d'actionnement, adaptée pour insertion d'instruments chirurgicaux et/ou de dispositifs à perception tactile,
- un dispositif support, pour supporter la surface d'actionnement.

7. Dispositif à perception tactile selon la revendication 6, dans lequel
l'élément d'actionnement (11) comprend une pluralité de trous (20) pour insertion d'outils chirurgicaux ou de dispositifs à perception tactile.

8. Dispositif à perception tactile selon la revendication 6, dans lequel l'élément d'actionnement (11) est conformé en tant que partie d'un corps humain.

9. Dispositif à perception tactile selon la revendication 6, dans lequel le dispositif à perception tactile comprend également un trocart (21), disposé de manière désolidarisable dans l'élément d'actionnement (11).

10. Dispositif à perception tactile selon la revendication 6, dans lequel la surface d'actionnement est conformée en tant que partie d'un corps humain.

11. Dispositif à perception tactile selon l'une des revendications 6 à 10, dans lequel la surface d'actionnement est une surface plane.

12. Dispositif à perception tactile selon l'une des revendications 7 à 11, dans lequel les trous (20) sont agencés en un motif.

13. Dispositif à perception tactile selon l'une des revendications 7 à 11, dans lequel les trous (20) sont agencés en une matrice.

14. Dispositif à perception tactile selon la revendication 6, dans lequel la surface d'actionnement est incurvée.

15. Dispositif à perception tactile selon l'une quelconque des revendications 6 à 14, dans lequel le dispositif support comprend des pieds.

16. Dispositif à perception tactile selon la revendication 15, dans lequel les pieds sont ajustables.

17. Dispositif à perception tactile selon l'une quelconque des revendications 6 à 16, dans lequel le dispositif support comprend des dispositifs de serrage, pour fixer l'élément d'actionnement à une table.

18. Dispositif à perception tactile selon l'une quelconque des revendications 7 à 17, dans lequel le dispositif à perception tactile comprend un trocart, agencé de façon désolidarisable sur certains des trous.

19. Dispositif à perception tactile selon l'une quelconque des revendications 6 à 18, dans lequel la surface d'actionnement comprend un tampon d'épaisseur.
